# EUROPEAN PATENT SPECIFICATION

(11) **EP 2 324 023 B1**
(45) Date of publication and mention of the grant of the patent: **17.08.2016**
(21) Application number: 07854812.0
(22) Date of filing: 28.11.2007
(51) Int. Cl.: A61K 31/535, A61Q 19/00

(54) **VIGOR ENHANCEMENT VIA ADMINISTRATION OF PYRIMIDINE DERIVATIVES**
VERSTÄRKUNG DER VITALITÄT DURCH VERABREICHUNG VON PYRIMIDINDERIVATEN
AMÉLIORATION DE LA VIGUEUR PAR ADMINISTRATION DE DÉRIVÉS DE PYRIMIDINE

(30) Priority: 28.11.2006 US 563891
(43) Date of publication of application: 25.05.2011
(73) Proprietor: University of Central Florida Research Foundation, Inc., Orlando FL 32826-3252 (US)
(72) Inventor: SUGAYA, Kiminobu, Winter Park FL 32792 (US); ALVAREZ, Angel, Chicago Illinois 60616 (US)
(74) Representative: Germinario, Claudio
(86) International application number: PCT/US2007/085765
(87) International publication number: WO 2009/070164

(56) References cited:
- WO-A1-2005/040391
- WO-A1-2006/009492
- WO-A1-2006/133876
- WO-A2-03/060082
- US-A- 5 304 555
- US-A- 5 976 523
- US-A1- 2003 113 912
- US-A1- 2003 139 410
- US-A1- 2006 003 919
- US-A1- 2006 147 435
- US-A1- 2006 257 449
- SANJO N ET AL: "A novel neurotrophic pyrimidine compound MS-818 enhances neurotrophic effects of basic fibroblast growth factor", JOURNAL OF NEUROSCIENCE RESEARCH, WILEY-LISS, US, vol. 54, 1 January 1998 (1998-01-01), pages 604-612, XP002971589, ISSN: 0360-4012, DOI: 10.1002/(SICI)1097-4547(19981201)54:5<604: :AID-JNR5>3.0.CO;2-W
- "MS-818", ANNUAL DRUG DATA REPORT, PROUS, BARCELONA, ES, vol. 19, no. 3, 1 January 1997 (1997-01-01), page 220, XP009129467, ISSN: 0379-4121
- LECHNER ET AL.: 'Stem/progenitor cells derived from adult tissues: potential for the treatment of diabetes mellitus' AM J PHYSIOL ENDOCRINOL METAB vol. 284, February 2003, pages E259 - E266, XP009056159

## Description

### Background

Much research is being conducted to study stem cells and to devise ways of utilizing stem cells in treating various neurological pathologies and injuries, as well as pathologies of other organ systems. It is generally recognized that stem cell technologies hold tremendous promise for ultimately treating and even curing neurologically related diseases, injuries or dysfunctions. It is understandable why stem cell research has focused on these areas. However, finding new and more diverse ways of utilizing stem cells is an ongoing challenge. It is important that stem cell research be directed to beneficial areas that do not necessarily include the traditional areas of developing treatments and cures for disease and injuries. Furthermore, agents that affect the characteristics of stem cells should be studied as this may reveal certain useful compounds that can be utilized to manipulate endogenous stem cells and thus leading to novel therapies.

### Detailed Description

The subject invention is based on the inventors' realization that relatively little research and development has been conducted in the area of stem cell-based applications for healthy individuals or subjects. Accordingly, the subject invention pertains to a method of increasing vigor of a human or nonhuman subject that comprises the administration of a vigor-enhancing composition that contains stem cell proliferating agent (SCPA). U.S. Patent Nos. 5,976,523 ('523 patent) and 4,959,368 ('368 patent) teach a number of compounds that may be used as wound healing agents. The '523 patent teaches that the wound healing agents described therein act by potentiating growth factors and cytokines released in tissues as a result of injury or wounding of tissues. Essentially, the '523 patent teaches that the agents stimulate the migration of cells toward the wound. The present inventors have discovered that the same agents actually stimulate the proliferation of stem cells, which in turn, led to the discovery that the agents may be used in circumstances where tissues have not been wounded.

Accordingly, the agents presented in the '523 patent and '368 patent for disclosure of SCPA agents. Formulas 1 and 2 as set forth in the '523 patent are provided: 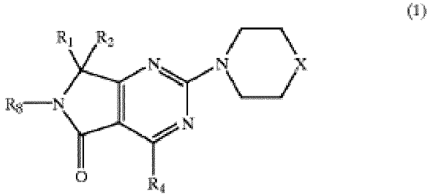 and

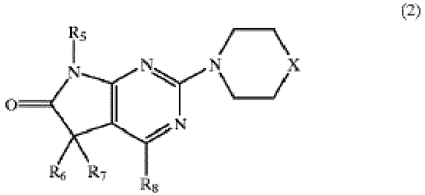

The present invention refers to the pyrimidine derivative of formula (I)MS-818, as disclosed in U.S. Pat. No. 4,959,368. In certain in vivo embodiments, the pyrimidine derivatives of the invention is administered at a concentration of between about 0.01 mg/kg/day to 50 mg/kg/day, more preferably between about 0.1 mg/kg/day to 10 mg/kg/day, even more preferably between about 1 mg/kg/day to 5 mg/kg/day, and even more preferably about 3 mg/kg/day. In these embodiments, the pyrimidine derivatives of the invention is administered for between about 1 and 60 days, or more preferably between about 1 and 30 days, or more preferably between about 1 and 15 days, or even more preferably between about 1 and 10 days, or more preferably between about 2 and 7 days, or even more preferably about 5 days. In certain others of these embodiments, the methods further comprise the step of administering a growth factor. In certain embodiments, the growth factor comprises fibroblast growth factor, epidermal growth factor or a combination thereof

*Increasing and*/*or Maintaining Vigor in a Subject*

In one embodiment, the subject invention pertains to a method of increasing vigor in a healthy subject comprising administering a therapeutically effective amount of a composition comprising a SCPA, wherein said agent is MS-818. As used herein, the term 'vigor' when used in the context of a subject's overall disposition without reference to administration to a tissue refers to the level of physical strength, stamina, energy, and/or mental strength of the subject; when used in the context of particular tissue(s) it refers to the level of juvenescence and robustness of the target tissue of the subject. Thus, an increase in vigor caused by administration of an SCPA may pertain to an increase in one or more of a subject's disposition characteristics or to specific tissue characteristics as compared to that where no administration of an SCPA is made. It is contemplated that the increase in vigor is not necessarily targeted to a pathology or disease. Accordingly, the methods taught herein can serve as a vigor boost to even a clinically healthy subject.

The active compound of certain composition embodiments, SCPA, may be included in a pharmaceutically acceptable carrier in an amount sufficient to exert a therapeutically useful effect in the absence of undesirable side effects on the patient treated. The therapeutically effective concentration may be determined empirically by testing the compounds in known in vitro and in vivo systems (see, e.g., Rosenthal et al. (1996) Antimicrob. Agents Chemother. 40(7):1600-1603; Dominguez et al. (1997) J. Med. Chem. 40:2726-2732; Clark et al. (1994) Molec. Biochem. Parasitol. 17:129; Ring et al. (1993) Proc. Natl. Acad. Sci. USA 90:3583-3587; Engel et al. (1998) J. Exp. Med. 188(4):725-734; Li et al. (1995) J. Med. Chem. 38:5031) and then extrapolated therefrom for dosages for humans.

The concentration of active compound in the pharmaceutical composition will depend on absorption, inactivation and excretion rates of the active compound, the physicochemical characteristics of the compound, the dosage schedule, and amount administered as well as other factors known to those of skill in the art.

Typically a therapeutically effective dosage should produce a serum concentration of active compound of from about 0.1 ng/ml to about 50-100 µg/ml. The pharmaceutical compositions typically should provide a dosage of from about 0.001 mg to about 2000 mg of compound per kilo-gram of body weight per day. Pharmaceutical dosage unit forms are prepared to provide from about 1 mg to about 1000 mg and preferably from about 10 to about 500 mg of the essential active ingredient or a combination of essential ingredients per dosage unit form.

The active compound may be administered at once, or may be divided into a number of smaller doses to be administered at intervals of time. It is understood that the precise dosage and duration of treatment is a function of the disease being treated and may be determined empirically using known testing protocols or by extrapolation from in vivo or in vitro test data. It is to be noted that concentrations and dosage values may also vary with the severity of the condition to be alleviated. It is to be further understood that for any particular subject, specific dosage regimens should be adjusted over time according to the individual need and the professional judgment of the person administering or supervising the administration of the compositions, and that the concentration ranges set forth herein are exemplary only and are not intended to limit the scope or practice of the claimed compositions.

Preferred pharmaceutically acceptable derivatives include acids, bases, enol ethers and esters, salts, esters, hydrates, solvates and prodrug forms. The derivative is selected such that its pharmacokinetic properties are superior to the corresponding neutral compound.
Thus, effective concentrations or amounts of one or more of the compounds described herein or pharmaceutically acceptable derivatives thereof are mixed with a suitable pharmaceutical carrier or vehicle for, topical or local administration to form pharmaceutical compositions. The concentration of active compound in the composition will depend on absorption, inactivation, excretion rates of the active compound, the dosage schedule, amount administered, particular formulation as well as other factors known to those of skill in the art.

In instances in which the compounds exhibit insufficient solubility, methods for solubilizing compounds may be used. Such methods are known to those of skill in this art, and include, but are not limited to, using cosolvents, such as dimethylsulfoxide (DMSO), using surfactants, such as TWEEN®, or dissolution in aqueous sodium bicarbonate. Derivatives of the compounds, such as prodrugs of the compounds may also be used in formulating effective pharmaceutical compositions.

Upon mixing or addition of the compound(s), the resulting mixture may be a solution, suspension, emulsion or the like. The form of the resulting mixture depends upon a number of factors, including the intended mode of administration and the solubility of the compound in the selected carrier or vehicle. The effective concentration is sufficient for ameliorating the symptoms of the disease, disorder or condition treated and may be empirically determined.

The pharmaceutical compositions are provided for administration to humans and animals in unit dosage forms, such as tablets, capsules, pills, powders, granules, sterile parenteral solutions or suspensions, and oral solutions or suspensions, and oil-water emulsions containing suitable quantities of the compounds or pharmaceutically acceptable derivatives thereof. The pharmaceutically therapeutically active compounds and derivatives thereof are typically formulated and administered in unit-dosage forms or multiple-dosage forms. Unit-dose forms as used herein refers to physically discrete units suitable for human and animal subjects and packaged individually as is known in the art. Each unit-dose contains a predetermined quantity of the therapeutically active compound sufficient to produce the desired therapeutic effect, in association with the required pharmaceutical carrier, vehicle or diluent. Examples of unit-dose forms include ampoules and syringes and individually packaged tablets or capsules. Unit-dose forms may be administered in fractions or multiples thereof. A multiple-dose form is a plurality of identical unit-dosage forms packaged in a single container to be administered in segregated unit-dose form. Examples of multiple-dose forms include vials, bottles of tablets or capsules or bottles of pints or gallons. Hence, multiple dose form is a multiple of unit-doses which are not segregated in packaging.

Certain composition embodiments can contain along with the active compound: a diluent such as lactose, sucrose, dicalcium phosphate, or carboxymethylcellulose; a lubricant, such as magnesium stearate, calcium stearate and talc; and a binder such as starch, natural gums, such as gum acaciagelatin, glucose, molasses, polvinylpyrrolidine, celluloses and derivatives thereof, povidone, crospovidones and other such binders known to those of skill in the art. Liquid pharmaceutically administrable compositions can, for example, be prepared by dissolving, dispersing, or otherwise mixing an active compound as defined above and optional pharmaceutical adjuvants in a carrier, such as, for example, water, saline, aqueous dextrose, glycerol, glycols, ethanol, and the like, to thereby form a solution or suspension. If desired, the pharmaceutical composition to be administered may also contain minor amounts of nontoxic auxiliary substances such as wetting agents, emulsifying agents, or solubilizing agents, pH buffering agents and the like, for example, acetate, sodium citrate, cyclodextrine derivatives, sorbitan monolaurate, triethanolamine sodium acetate, triethanolamine oleate, and other such agents. Actual methods of preparing such dosage forms are known, or will be apparent, to those skilled in this art; for example, see Remington's Pharmaceutical Sciences, Mack Publishing Company, Easton, Pa., 15th Edition, 1975. The composition or formulation to be administered will, in any event, contain a quantity of the active compound in an amount sufficient to alleviate the symptoms of the treated subject.

Dosage forms or compositions containing active ingredient in the range of 0.005% to 100% with the balance made up from non-toxic carrier may be prepared. For oral administration, a pharmaceutically acceptable non-toxic composition is formed by the incorporation of any of the normally employed excipients, such as, for example pharmaceutical grades of mannitol, lactose, starch, magnesium stearate, talcum, cellulose derivatives, sodium crosscarmellose, glucose, sucrose, magnesium carbonate or sodium saccharin. Such compositions include solutions, suspensions, tablets, capsules, powders and sustained release formulations, such as, but not limited to, implants and microencapsulated delivery systems, and biodegradable, biocompatible polymers, such as collagen, ethylene vinyl acetate, polyanhydrides, polyglycolic acid, polyorthoesters, polylactic acid and others. Methods for preparation of these compositions are known to those skilled in the art. The contemplated compositions may contain 0.001%-100% active ingredient, preferably 0.1-85%, typically 75-95%.

The active compounds or pharmaceutically acceptable derivatives may be prepared with carriers that protect the compound against rapid elimination from the body, such as time release formulations or coatings.

Of interest herein are also lyophilized powders, which can be reconstituted for administration as solutions, emulsions and other mixtures. They may also be reconstituted and formulated as solids or gels. The sterile, lyophilized powder is prepared by dissolving a SCPA compound in a suitable solvent. The solvent may contain an excipient which improves the stability or other pharmacological component of the powder or reconstituted solution, prepared from the powder. Excipients that may be used include, but are not limited to, dextrose, sorbital, fructose, corn syrup, xylitol, glycerin, glucose, sucrose or other suitable agent. The solvent may also contain a buffer, such as citrate, sodium or potassium phosphate or other such buffer known to those of skill in the art at, typically, about neutral pH. Subsequent sterile filtration of the solution followed by lyophilization under standard conditions known to those of skill in the art provides the desired formulation. Generally, the resulting solution will be apportioned into vials for lyophilization. Each vial will contain a single dosage (10-1000 mg, preferably 100-500 mg) or multiple dosages of the compound. The lyophilized powder can be stored under appropriate conditions, such as at about 4° C. to room temperature.

Reconstitution of this lyophilized powder with water for injection provides a formulation for use in parenteral administration. For reconstitution, about 1-50 mg, preferably 5-35 mg, more preferably about 9-30 mg of lyophilized powder, is added per mL of sterile water or other suitable carrier. The precise amount depends upon the selected compound. Such amount can be empirically determined.

Topical mixtures are prepared as described for the local and systemic administration. The resulting mixture may be a solution, suspension, emulsions or the like and are formulated as creams, gels, ointments, emulsions, solutions, elixirs, lotions, suspensions, tinctures, pastes, foams, aerosols, irrigations, sprays, suppositories, bandages, dermal patches or any other formulations suitable for topical administration.

The compounds or pharmaceutically acceptable derivatives thereof may be formulated as aerosols for topical application, such as by inhalation (see, e.g., U.S. Pat. Nos. 4,044,126, 4,414,209, and 4,364,923, which describe aerosols for delivery of a steroid useful for treatment inflammatory diseases, particularly asthma). These formulations for administration to the respiratory tract can be in the form of an aerosol or solution for a nebulizer, or as a microfine powder for insufflation, alone or in combination with an inert carrier such as lactose. In such a case, the particles of the formulation will typically have diameters of less than 50 microns, preferably less than 10 microns.

The compounds may be formulated for local or topical application, such as for topical application to the skin and mucous membranes, such as in the eye, in the form of gels, liquid drops, creams, and lotions and for application to the eye or for intracisternal or intraspinal application. Topical administration is contemplated for transdermal delivery and also for administration to the eyes or mucosa, or for inhalation therapies. Nasal solutions of the active compound alone or in combination with other pharmaceutically acceptable excipients can also be administered.

These solutions, particularly those intended for ophthalmic use, may be formulated as 0.01%-10% isotonic solutions, pH about 5-7, with appropriate salts.

In a specific embodiment, the subject invention pertains to a method of increasing vigor of a subject's skin comprising topically administering a therapeutically effective amount of a composition comprising a SCPA according to a regimen by which said skin vigor is increased. Increased skin vigor may pertain to improved skin tone, diminishing of wrinkles, improved complexion, improved skin smoothness, improved, skin resiliency, flexibility and/or elasticity or other characteristics representing increased juvenescence or appearance thereof, of the skin of the subject. In another specific embodiment, the subject invention pertains to a method of maintaining vigor of a subject's skin comprising a comprising topically administering a therapeutically effective amount of a composition comprising a SCPA according to a regimen by which said skin vigor is maintained over a period of time. A regimen may pertain to a certain amount, applied according to a certain frequency over a certain period of time.

According to certain embodiments of the topical compositions of the present invention also includes at least one of the following: a surface smoother, a skin plumper, an optical diffuser, a sunscreen, an exfoliation promoter, and an antioxidant.
(i) A surface smoother provides the functional benefits of enhancing skin smoothness and reducing the appearance of fine lines and coarse wrinkles. Examples include silicas, talcs, isopropyl myristate, petrolatum, isopropyl lanolate, silicones (e.g., methicone, dimethicone), polymethylmethacrylate (PMMA), or any mixtures thereof. The surface smoother is preferably present from about 0.1 wt % to about 50 wt % of the total weight of the composition.
(ii) A skin plumper serves as a collagen enhancer to the skin. An example of a suitable, and preferred, skin plumper is palmitoyl oligopeptide. Other skin plumpers are collagen and/or glycosaminoglycan (GAG) enhancing agents. The skin plumper is preferably present from about 0.1 wt % to about 20 wt % of the total weight of the composition.
(iii) An optical diffuser is a particle that changes the surface optometrics of skin, resulting in a visual blurring and softening of, for example, lines and wrinkles. Examples of optical diffusers that can be used in the present invention include, but are not limited to, boron nitride, mica, nylon, polyurethane powder, sericite, silica, silicone powder, talc, Teflon, titanium dioxide, zinc oxide, or any mixtures thereof. The optical diffuser is preferably present from about 0.01 wt % to about 20 wt % of the total weight of the composition.
(iv) A sunscreen protects the skin from damaging ultraviolet rays. In an illustrative embodiment of the present invention, the sunscreen would provide both UVA and UVB protection, by using either a single sunscreen or a combination of sunscreens. Among the sunscreens that can be employed in the present compositions are avobenzone, cinnamic acid derivatives (such as octylmethoxy cinnamate), octyl salicylate, oxybenzone, titanium dioxide, zinc oxide, or any mixtures thereof. Preferably, the sunscreen is present from about 1 wt % to about 30 wt % of the total weight of the composition. In particular, the addition of a sunscreen is preferred to prevent/reduce the photodegradation of retinoid while in the package and/or on the skin.

Other routes of administration, such as transdermal patches and rectal administration are also contemplated herein. For example, pharmaceutical dosage forms for rectal administration are rectal suppositories, capsules and tablets for systemic effect. Rectal suppositories are used herein mean solid bodies for insertion into the rectum which melt or soften at body temperature releasing one or more pharmacologically or therapeutically active ingredients. Pharmaceutically acceptable substances utilized in rectal suppositories are bases or vehicles and agents to raise the melting point. Examples of bases include cocoa butter (theobroma oil), glycerin-gelatin, carbowax (polyoxyethylene glycol) and appropriate mixtures of mono-, di- and triglycerides of fatty acids. Combinations of the various bases may be used. Agents to raise the melting point of suppositories include spermaceti and wax. Rectal suppositories may be prepared either by the compressed method or by molding. The typical weight of a rectal suppository is about 2 to 3 gm.

Tablets and capsules for rectal administration are manufactured using the same pharmaceutically acceptable substance and by the same methods as for formulations for oral administration.

The SCPA compounds or pharmaceutically acceptable derivatives may be packaged as articles of manufacture containing packaging material, a compound or pharmaceutically acceptable derivative thereof provided herein.

The articles of manufacture provided herein contain packaging materials. Packaging materials for use in packaging pharmaceutical products are well known to those of skill in the art. See, e.g., U.S. Pat. Nos. 5,323,907, 5,052,558 and 5,033,352. Examples of pharmaceutical packaging materials include, but are not limited to, blister packs, bottles, tubes, inhalers, pumps, bags, vials, containers, syringes, bottles, and any packaging material suitable for a selected formulation and intended mode of administration and treatment. A wide array of formulations of the compounds and compositions provided herein are contemplated for treatment and prevention of insulin resistance.

## Claims

1. A method for increasing vigor of a healthy subject's skin comprising topical administering an effective amount of a SCPA of formula (1), wherein the SCPA is compound MS-818.

2. The method according to claim 1, wherein increasing skin vigor comprises improving skin tone, diminishing wrinkles, improving complexion, improving skin smoothness, improving skin resiliency, improving skin flexibility and/or elasticity.

3. The method according to claim 1 or 2, wherein said compound MS-818 is in a composition comprising a surface smoother, a skin plumper, an optical diffuser, a sunscreen, an exfoliation promoter, or an antioxidant, or a combination thereof.

## Patentansprüche

1. Verfahren zum Verstärken der Vitalität der Haut einer gesunden Person, das ein topisches Verabreichen einer effektiven Menge eines SCPA nach Formel (1) umfasst, wobei das SCPA Verbindung MS-818 ist.

2. Verfahren nach Anspruch 1, wobei ein Verstärken der Hautvitalität umfasst:
Verbessern des Hauttones, Verringern von Falten, Verbessern des Hautbildes, Verbessern der Hautglätte, Verbessern der Hautspannung, Verbessern der Hautflexibilität und/oder -elastizität.

3. Verfahren nach Anspruch 1 oder 2, wobei die Verbindung MS-818 sich in einer Zusammensetzung befindet, die einen Oberflächenglätter, einen optischen Diffusor, ein Peeling, ein Antioxidationsmittel oder eine Kombination davon enthält.

## Revendications

1. Méthode destinée à accroître la vigueur de la peau d'un sujet sain comprenant l'administration topique d'une quantité efficace d'un SCPA de formule (1), dans laquelle le SCPA est un composé MS-818.

2. Méthode selon la revendication 1, dans laquelle l'accroissement de la vigueur de la peau comprend l'amélioration de la couleur de la peau, la diminution des rides, l'amélioration du teint, l'amélioration du lisseur de la peau, l'amélioration de la résilience de la peau, l'amélioration de la souplesse et/ou de l'élasticité de la peau.

3. Méthode selon la revendication 1 ou 2, dans laquelle ledit composé MS-818 fait partie d'une composition comprenant un agent de relissage cutané, un repulpeur de peau, un diffuseur optique, un écran solaire, un agent favorisant l'exfoliation, ou un antioxydant, ou une combinaison de ceux-ci.
